(19)

Europäisches Patentamt

European Patent Office

Office européen des brevets

(11) **EP 2 737 854 A1**

(12) **EUROPEAN PATENT APPLICATION**
published in accordance with Art. 153(4) EPC

(43) Date of publication:
**04.06.2014 Bulletin 2014/23**

(21) Application number: **12818406.6**

(22) Date of filing: **05.07.2012**

(51) Int Cl.:
*A61B 6/03* (2006.01)      *G06T 1/00* (2006.01)

(86) International application number:
**PCT/JP2012/004378**

(87) International publication number:
**WO 2013/014868 (31.01.2013 Gazette 2013/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.07.2011   JP 2011165453**

(71) Applicant: **Panasonic Healthcare Co., Ltd. Ehime 791-0395 (JP)**

(72) Inventors:
• **IMANAKA, Ryoichi**
  **Osaka 540-6207 (JP)**

• **KOHYAMA, Tsuyoshi**
  **Osaka 540-6207 (JP)**
• **TAKEMURA, Tomoaki**
  **Osaka 540-6207 (JP)**
• **IMANISHI, Keiho**
  **Hyogo 663-8179 (JP)**

(74) Representative: **Eisenführ Speiser Patentanwälte Rechtsanwälte PartGmbB Postfach 10 60 78 28060 Bremen (DE)**

(54) **CUTTING SIMULATION DEVICE AND CUTTING SIMULATION PROGRAM**

(57)   A PC (1) comprises a tomographic image information acquisition section (6), a memory (9), a volume rendering calculator (13), a display unit (2), a mouse (4), and a voxel label setting section (18). The tomographic image information acquisition section (6) acquires tomographic image information. The memory (9) stores voxel information about the tomographic image information. The volume rendering calculator (13) samples voxel information in a direction perpendicular to the line of sight, on the basis of the voxel information. The display unit (2) displays calculation results from the volume rendering calculator (13). The mouse (4) inputs cutting instructions for a liver (22) displayed on the display unit (2). The voxel label setting section (18) displays the status of the liver (22) after cutting using the cutting instructions from the mouse (4), and displays blood vessels (23) inside the liver (22) included in a cut portion (C), in a pre-cut state even after cutting.

FIG. 2

EP 2 737 854 A1

**Description**

TECHNICAL FIELD

The present invention relates to a cutting simulation device and a cutting simulation program utilized when a healthcare worker performs a surgery simulation.

BACKGROUND ART

Cutting simulation devices that allow simulations of surgery to be performed are utilized at healthcare sites to improve the quality of surgery.

A conventional cutting simulation device comprised, for example, a tomographic image information acquisition section for acquiring tomographic image information, such as an X-ray CT image, a nuclear magnetic resonance image (MRI), or an image acquired by PET (positive electron tomography), a memory connected to the tomographic image information acquisition section, a volume rendering calculator connected to the memory, a display for displaying the calculation results of the volume rendering calculator, and an input section for being inputted cutting instructions with respect to the display object that is displayed on the display.

Patent Literature 1, for example, discloses a surgery simulation system in which cutting instructions for a display object that is displayed in 3D on the display are given by an input section.

Patent Literature 2 discloses an image processing device with which the site to be viewed can be properly displayed by displaying other structures semitransparently, even when the site to be viewed in a 3D display (such as a tumor) is hidden by other structures (such as blood vessels).

CITATION LIST

PATENT LITERATURE

Patent Literature 1: Japanese Laid-Open Patent Application H5-123327
Patent Literature 2: Japanese Laid-Open Patent Application 2003-91735

SUMMARY

TECHNICAL PROBLEM

However, the following problems were encountered with the conventional configurations discussed above.

With the image processing device disclosed in Patent Literature 2, the shape of a particular region of a tumor or the like can be recognized from all directions, but nothing at all is disclosed about display control when this is to be cut.

Thus, when cutting was performed on an organ or other such cutting object with the surgery simulation system of Patent Literature 1 on a 3D display produced by the image processing device of Patent Literature 2, the display could only show a state in which the blood vessels, nerves, and the like present inside the organ were also cut. Therefore, a physician or the like performing the cutting simulation cannot share information that would be beneficial in real surgery, and there is the risk that an effective surgery simulation cannot be carried out.

It is an object of the present invention to provide a cutting simulation device and a cutting simulation program with which an actual surgical process can be represented in detail by visualizing the blood vessels, nerves, and other such non-cutting objects present inside an organ or other such cutting object even after cutting.

SOLUTION TO PROBLEM

The cutting simulation device pertaining to the first invention comprises a tomographic image information acquisition section, a memory, a volume rendering calculator, a display unit, an input section, and a display controller. The tomographic image information acquisition section acquires tomographic image information. The memory is connected to the tomographic image information acquisition section and stores voxel information about the tomographic image information. The volume rendering calculator is connected to the memory and samples voxel information in a direction perpendicular to the line of sight, on the basis of the voxel information. The display unit displays calculation results from the volume rendering calculator. The input section is inputted with instructions for cutting a cutting object displayed on the display unit. The display controller displays on the display unit the status of the cutting object after cutting on the basis of the cutting instructions inputted with the input section, and displays non-cutting objects included inside the cutting object, on the display unit in a pre-cut state even after cutting, even when included in a cut portion according to the cutting instructions.

Here, when conducting a cutting simulation using a plurality of X-ray CT images in a state of a 3D display of the area around a particular organ, for example, if the portion to be cut includes both the particular organ (the cutting object) and the blood vessels and so forth in the inside of the organ (non-cutting objects), then control is performed so that only the particular organ that is the cutting object is cut, while the blood vessels or other non-cutting objects are still displayed in their pre-cut state.

The above-mentioned tomographic image includes two-dimensional images acquired using a medical device, such as an X-ray CT, an MRI, or a PET. Also, the relation between the cutting object and the non-cutting objects includes, for example, the relation between an organ and the blood vessels, nerves, and so forth present on the inside of the organ.

Consequently, in conducting a simulation of cutting part of a 3D displayed organ, in the past, the display was post-cutting, in a state in which blood vessels, nerves, and other such tissue inside the organ had been cut at the same time as the cutting of the organ, whereas with the present invention, only the organ that is the cutting object is cut, and the blood vessels or the like that are the non-cutting objects present inside the organ are displayed in their pre-cutting state.

Thus, before a physician or the like performs a surgical operation, the positions of blood vessels and so on in the cut portion of the 3D image including the actual surgical site on the patient can be shared among a plurality of physicians, etc. As a result, the actual surgical procedure can be displayed as a detailed simulation.

The cutting simulation device pertaining to the second invention is the cutting simulation device pertaining to the first invention, wherein if non-cutting objects are included in the cut portion, the display controller switches the voxel data for the cut portion with voxel data for the non-cutting objects, and displays the result on the display unit.

Here, in post-cutting display control during the simulation, the voxel data for the cut portion and the voxel data for the non-cutting objects are switched in the display for the non-cutting objects included in the cut portion.

Consequently, for the cutting object included in the cut portion, a state is displayed in which the cut processing is left as it is, but for the non-cutting objects included in the cut portion, their pre-cutting state is displayed. Thus, the physician, etc., can perform an actual operation while correctly recognizing the positions of the blood vessels, nerves, and other such non-cutting objects included in the cut portion.

The cutting simulation device pertaining to the third invention is the cutting simulation device pertaining to the first or second invention, wherein color information is added to the voxel information corresponding to the cutting object and the non-cutting objects in display on the display unit. The display controller displays the non-cutting objects on the display unit in a different color from the color of the cutting object.

Here, the objects are displayed in different colors, such as blood vessels and other such non-cutting objects in red, and organs and other such cutting objects in reddish-brown, in order to make it easier to recognize the non-cutting objects displayed even after the cut processing of the cut portion apart from the cutting object displayed in a cut state.

Consequently, a physician, etc., conducting the simulation can more clearly recognize whether or not there are any blood vessels or the like that would be non-cutting objects in the cut portion of the organ that is the cutting object.

The cutting simulation device pertaining to the fourth invention is the cutting simulation device pertaining to any of the first to third inventions, wherein a range of CT values indicating the extent of spatial X-ray absorption during X-ray irradiation of a subject is set for the cutting object and the non-cutting objects. The display controller displays the desired cutting object and non-cutting objects on the display unit on the basis of the CT values inputted via the input section.

Here, for organs or the like (the cutting object) and blood vessels, nerves, or the like (the non-cutting objects) each have a designated, the objects (cutting object and non-cutting objects) displayed on the display unit are switched by presetting range of CT values.

The CT value mentioned here is the product of expressing the extent of X-ray absorption in a human body as a numerical value, and is expressed as a relative value (units: HU) versus a zero for water. For example, the CT value for a liver is from 60 to 70 HU, the CT value for a kidney is from 30 to 40 HU, the CT value for blood is from 30 to 50 HU, and the CT value for bone is from 500 to 1000 HU.

Consequently, by using a mouse, keyboard, or other such input section to input a range of CT values for the organ, etc., on which the surgery is actually to be performed, the desired organ, etc., can be easily displayed on the display unit, and the above-mentioned cutting simulation carried out.

The cutting simulation device pertaining to the fifth invention is the cutting simulation device pertaining to any of the first to fourth inventions, wherein the cutting object includes an organ or bone.

Here, a liver, a kidney, a pancreas, a duodenum, a stomach, a large intestine, a small intestine, or another such organ or a bone is used as the cutting object that will be the object of the above-mentioned cutting simulation.

Consequently, the above-mentioned cutting simulation can be performed for the desired organ or bone which needs a surgical operation.

The cutting simulation device pertaining to the sixth invention is the cutting simulation device pertaining to any of the first to fifth inventions, wherein the non-cutting objects include blood vessels or nerves.

Here, blood vessels, nerves, or the like present inside the organ serving as the cutting object are used, for example, as the non-cutting objects on which the above-mentioned cutting simulation will be performed.

Consequently, where the blood vessels, etc., included in the cut portion of the organ, etc., needing surgery are located can be clearly recognized.

The cutting simulation program pertaining to the seventh invention causes a computer to execute a cutting simulation method comprising an acquisition step, a volume rendering step, a first display step, and a second display step. The acquisition step involves acquiring tomographic image information. The volume rendering step involves sampling voxel information in a direction perpendicular to the line of sight, on the basis of voxel information about the tomographic image information. The first display step involves displaying calculation results from the volume rendering. The second display step involves displaying on the display unit the status of the cutting object after cutting on the basis of the cutting instructions inputted with respect to the displayed cutting object, and displaying non-cutting objects included inside the cutting object, on the display unit in a pre-cut state even after cutting, even when included in a cut portion according to the cutting instructions.

Here, in conducting a cutting simulation in a state in which a plurality of X-ray CT images are used to create a 3D display of the area around a particular organ, for example, if the portion to be cut includes both the particular organ (the cutting object) and the blood vessels and so forth in the inside of the organ (non-cutting objects), then control is performed so that only the particular organ that is the cutting object is cut, while the blood vessels or other non-cutting objects are still displayed in their pre-cut state.

The above-mentioned tomographic image includes images acquired using a medical device, such as an X-ray CT, an MRI, or a PET. Also, the relation between the cutting object and the non-cutting objects includes, for example, the relation between an organ and the blood vessels, nerves, and so forth present on the inside of the organ.

Consequently, in conducting a simulation of cutting part of a 3D displayed organ, in the past, the display was post-cutting, in a state in which blood vessels, nerves, and other such tissue inside the organ had been cut at the same time as the cutting of the organ, whereas with the present invention, only the organ that is the cutting object is cut, and the blood vessels or the like that are the non-cutting objects present inside the organ are displayed in their pre-cutting state.

Thus, before a physician or the like performs a surgical operation, the positions of blood vessels and so on in the cut portion of the 3D image including the actual surgical site on the patient can be shared among a plurality of physicians, etc. As a result, the actual surgical process can be displayed as a detailed simulation.

ADVANTAGEOUS EFFECTS

With the cutting simulation device pertaining to the present invention, an actual surgical procedure can be represented in detail by visualizing the blood vessels, nerves, and so forth present inside a cutting object.

BRIEF DESCRIPTION OF DRAWINGS

FIG. 1 is an oblique view of a personal computer (cutting simulation device) pertaining to an embodiment of the present invention;
FIG. 2 is a control block diagram of the personal computer in FIG. 1;
FIG. 3 is a block diagram of the configuration of a voxel label storage section in a memory included in the control blocks in FIG. 2;
FIG. 4 is a block diagram of the configuration of a color information storage section in the memory included in the control blocks in FIG. 2;
FIGS. 5a and 5b are operational flowcharts of the personal computer in FIG. 1;
FIG. 6 is a concept diagram illustrating the operation of the personal computer in FIG. 1; and
FIG. 7a is a reference diagram showing an example of a post-cutting display image displayed on a typical cutting simulation device, FIG. 7b is a diagram showing an example of blood vessels around a liver, and FIG. 7c is a diagram showing an example of a post-cutting display image displayed on the personal computer in FIG. 1.

DESCRIPTION OF EMBODIMENTS

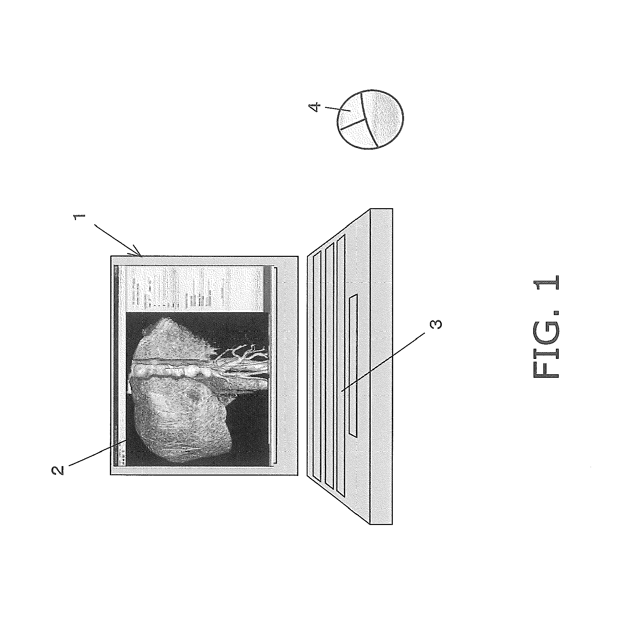

The personal computer (cutting simulation device) pertaining to an embodiment of the present invention will now be described through reference to FIGS. 1 to 7.

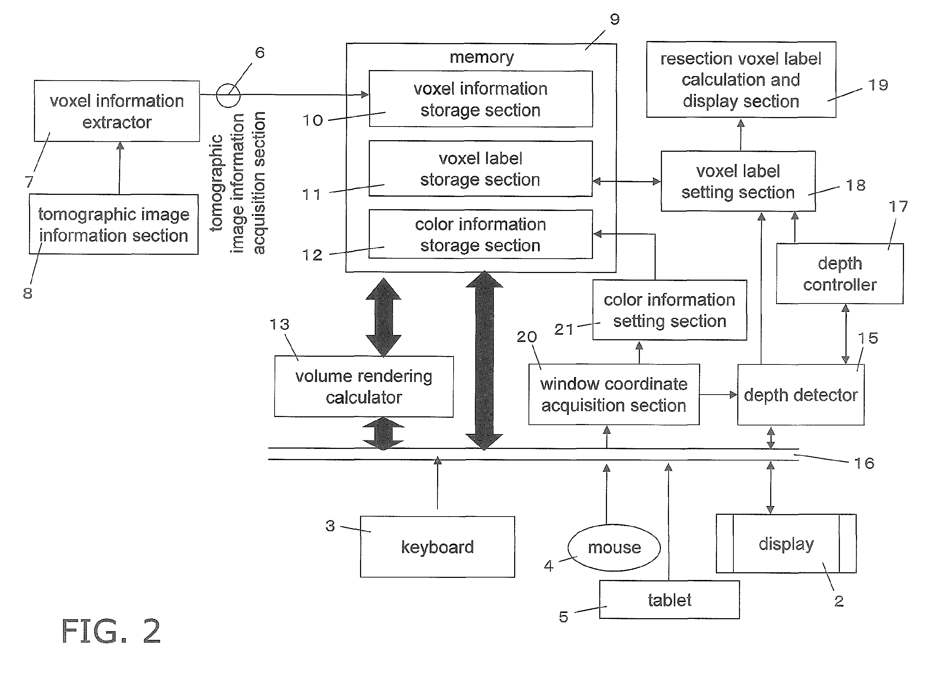

As shown in FIG. 1, the personal computer 1 pertaining to this embodiment comprises a display (display unit) 2 and various kinds of input section (a keyboard 3, a mouse 4, and a tablet 5 (see FIG. 2)).

The display 2 shows a 3D image of an organ or the like (a kidney is displayed in the example in FIG. 1) formed from a plurality of tomographic images such as X-ray CT images, and shows the results of a cutting simulation (discussed below).

As shown in FIG. 2, the personal computer 1 has internal control blocks such as a tomographic image information acquisition section 6.

The tomographic image information acquisition section 6 is connected to a tomographic image information section 8 via a voxel information extractor 7. That is, at the tomographic image information section 8, tomographic image information is supplied from a device that captures tomographic images, such as a CT, an MRI, or a PET, and this tomographic image information is extracted as voxel information by the voxel information extractor 7.

A memory 9 is provided inside the personal computer 1, and has a voxel information storage section 10, a voxel label storage section 11, and a color information storage section 12. The memory 9 is connected to a volume rendering calculator 13.

The voxel information storage section 10 holds voxel information received from the voxel information extractor 7 via the tomographic image information acquisition section 6.

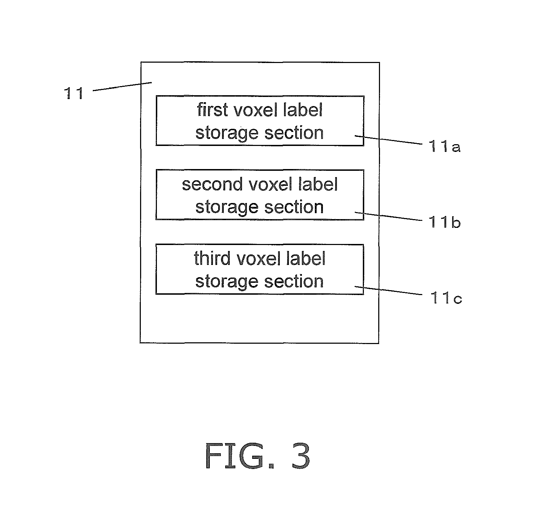

As shown in FIG. 3, the voxel label storage section 11 has a first voxel label storage section 11a, a second voxel label storage section 11b, and a third voxel label storage section 11c. These first to third voxel label storage sections 11a to 11c are each provided corresponding to a range of preset CT values (discussed below), that is, to the organs that will be displayed. For instance, the first voxel label storage section 11a corresponds to a range of CT values displaying a liver, the second voxel label storage section 11b corresponds to a range of CT values displaying blood vessels, and the third voxel label storage section 11c corresponds to a range of CT values displaying bone.

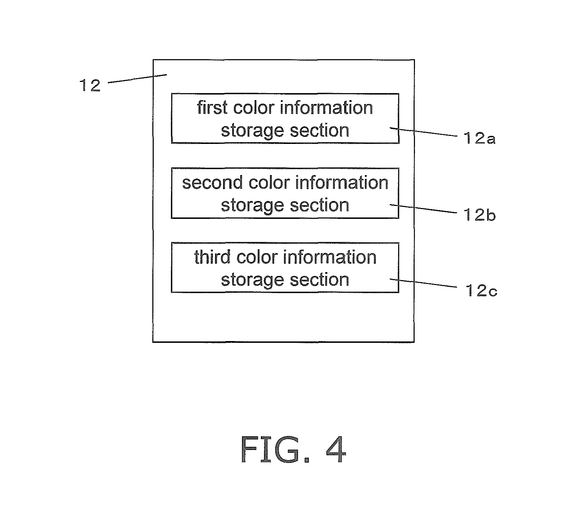

As shown in FIG. 4, the color information storage section 12 has a first color information storage section 12a, a second color information storage section 12b, and a third color information storage section 12c. These first to third color information storage sections 12a to 12c are similar to the first to third voxel label storage sections 11a to 11c in that they are each provided corresponding to a range of preset CT values (discussed below), that is, to the organs that will be displayed. For instance, the first color information storage section 12a corresponds to a range of CT values displaying a liver, the second color information storage section 12b corresponds to a range of CT values displaying blood vessels, and the third color information storage section 12c corresponds to a range of CT values displaying bone. The first to third color information storage sections 12a to 12c are each set to different color information for the organ, blood vessels, or bone to be displayed. For instance, reddish-brown color information is held in the range of CT values corresponding to a liver, red color information is held in the range of CT values corresponding to blood vessels, and white color information is held in the range of CT values corresponding to bone.

The CT values set for the organ, blood vessels, or bone to be displayed are the product of expressing the extent of X-ray absorption in a human body as a numerical value, and are expressed as relative values (units: HU) versus a zero for water. For example, the range of CT values in which a liver is displayed is from 60 to 70 HU, the range of CT values in which a kidney is displayed is from 30 to 40 HU, the range of CT values in which blood is displayed is from 30 to 50 HU, and the range of CT values in which bone is displayed is from 500 to 1000 HU.

The volume rendering calculator 13 acquires information about a plurality of slices at regular intervals in the Z direction and perpendicular to the line of sight, on the basis of the voxel information stored in the voxel information storage section 10, the voxel label stored in the voxel label storage section 11 and the color information stored in the color information storage section 12. The volume rendering calculator 13 then displays this calculation result as a 3D image on the display 2. The volume rendering calculator 13 is connected to a depth detector 15 via a bus 16.

The depth detector 15 measures a ray casting scanning distance (discussed below), and is connected to a depth controller 17 and a voxel label setting section 18.

The voxel label setting section 18 is connected to the voxel label storage section 11 and a resection voxel label calculation and display unit 19.

In addition to the above-mentioned volume rendering calculator 13 and depth detector 15, the bus 16 is connected to the color information storage section 12 and a window coordinate acquisition section 20, and displays 3D images and the like on the display 2 on the basis of what is inputted from the keyboard 3, the mouse 4, the tablet 5, etc.

The window coordinate acquisition section 20 is connected to the depth detector 15 and a color information setting section 21. The color information setting section 21 is connected to the color information storage section 12 in the memory 9.

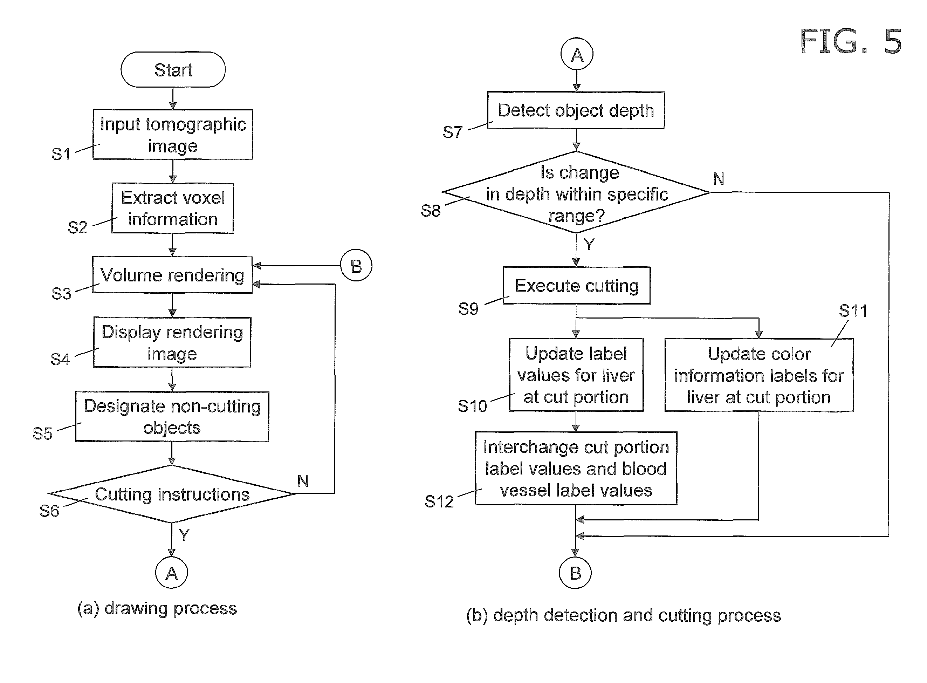

FIGS. 5a and 5b are control flowcharts illustrating the operation of the cutting simulation device in this embodiment.

As shown in FIG. 5a, with the personal computer 1 in this embodiment, first, in step S1, as mentioned above, tomographic image information is inputted from the tomographic image information section 8, and this is supplied to the voxel information extractor 7.

Next, in S2, voxel information is extracted from the tomographic image information by the voxel information extractor 7. The extracted voxel information is stored via the tomographic image information acquisition section 6 in the voxel information storage section 10 of the memory 9. The voxel information stored in the voxel information storage section 10 is information about the points that make up I (x, y, z, $\alpha$), for example. I here is brightness information for said points, x, y, and z are coordinate points, and $\alpha$ is transparency information.

Next, in S3, the volume rendering calculator 13 calculates information about a plurality of slices that are perpendicular to the line of sight and are regularly spaced, on the basis of voxel information stored in the voxel information storage section 10, and acquires a slice information group. The slice information group is then stored, at least temporarily, in the volume rendering calculator 13.

The above-mentioned "information about slices perpendicular to the line of sight" means a plane that is at a right angle to the line of sight. For instance, in a state in which the display 2 is stood up vertically and viewed in a state of being parallel to the viewer's face, the slice information is in a plane that is perpendicular to the line of sight.

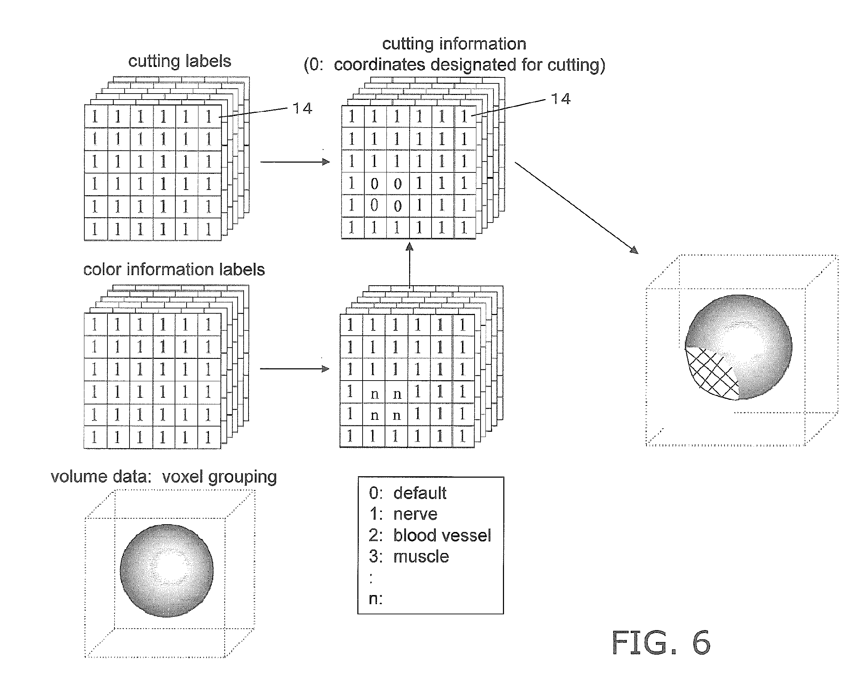

The information about a plurality of slices thus obtained includes information about the points constituted by I (x, y, z, $\alpha$), as mentioned above. Thus, the slice information comprises a plurality of voxel labels 14 disposed in the Z direction as shown in FIG. 6, for example. The grouping of voxel labels 14 shown in FIG. 6 is stored in the voxel label storage section 11, for example.

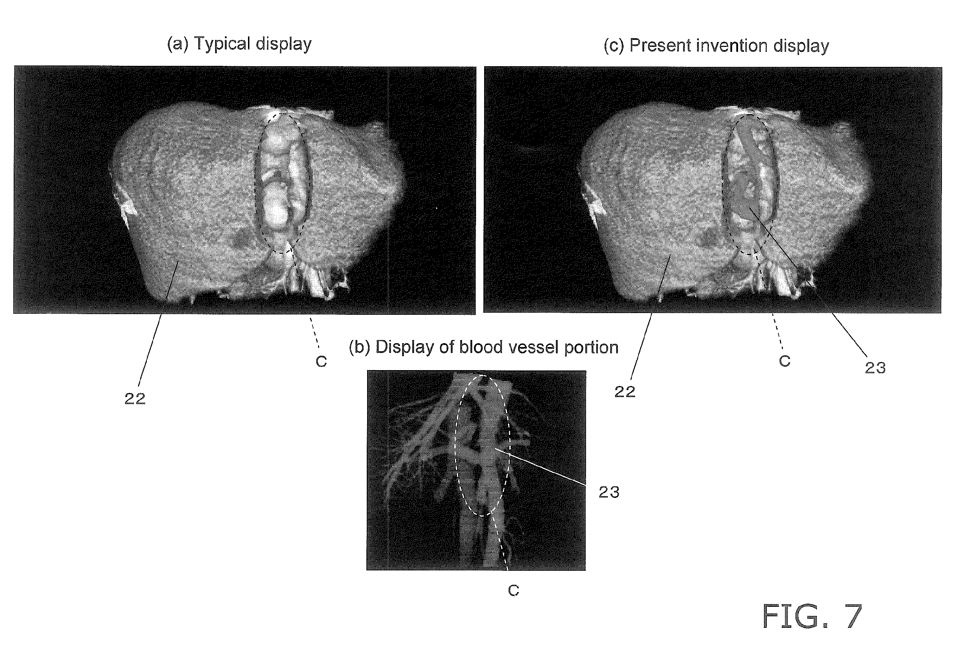

Next, in S4, a rendering image is displayed on the display 2. On the display 2 at this point an organ that will serve as the cutting object (a liver 22 in this embodiment) is selected by using the mouse 4 or the like to designate a range of CT values, and this is displayed as shown in FIG. 7a, etc.

In FIGS. 7a to 7c, 22 is a kidney, 23 is blood vessels, and C is the cut portion (discussed below). That is, in this embodiment, we will assume that a simulation of surgery on the liver 22 is to be performed.

As shown in FIG. 7a, when a typical cutting simulation is performed, the display 2 displays the state after part of the liver 22 or part of the blood vessels 23 included in the cut portion C has been cut down to a specific depth.

In this embodiment, in performing this cutting simulation of the liver 22, control is performed as follows so that where in the cut portion C the blood vessels 23 shown in FIG. 7b are present can be displayed on the screen of the display 2 in performing surgery to resect part of the liver 22.

Specifically, in S5, the mouse 4, etc., is used to set the CT values corresponding to the organ or the like that will be the cutting object in performing the cutting simulation, and the CT values corresponding to the blood vessels 23 or the like that will be the non-cutting objects. This setting of the cutting object and non-cutting objects may be accomplished by using the keyboard 3, the mouse 4, the tablet 5, or anything else.

Next, in S6, the mouse 4, etc., is used to give a cutting instruction. Just as with the setting of the cutting object and non-cutting objects, the input section used to input the cutting instructions may be the keyboard 3, the mouse 4, the tablet 5, or anything else.

As a specific method for inputting a cutting instruction, the mouse 4 is moved horizontally over a desktop so that the cursor displayed on the display 2 moves up and down, or to the left and right, over the liver 22.

The left/right or up/down movement of the mouse 4 here is detected by the window coordinate acquisition section 20. This information is transmitted through the depth detector 15 to the voxel label setting section 18 and the voxel label storage section 11. Consequently, cutting is performed that takes into account the positions of the liver 22 and the blood vessels 23 in the Z direction.

More specifically, the volume rendering calculator 13 samples voxel information at regular intervals in a direction perpendicular to the line of sight (this is called ray casting). The volume rendering calculator 13 then uses the depth detector 15 in S7 to detect the ray casting scanning distance (depth) for all the points found during movement of the mouse 4.

Then, in S8, it is determined whether or not the proportional change in this depth is within a specific range.

More specifically, the ray casting scanning distances d measured by the depth detector 15 are tabulated, and the gradient $\nabla d$ thereof is calculated. The gradient $\nabla d$ is compared with a threshold T to determine whether or not cutting needs to be executed. For example, if a gradient $\nabla d_i$ at a cutting point $p_i$ is over a threshold $T_i$, the cutting point is deemed invalid, and no cutting is performed. On the other hand, if the gradient $\nabla d_i$ at the

cutting point $p_i$ is within the threshold $T_i$, the cutting point is deemed valid, and cutting is performed in S9.

As to the threshold T, the threshold $T_i$ is determined on the basis of a multiple coefficient m and gradient average for n number of cutting points in the immediate vicinity for each cutting processing.

$$\mathbf{T}_i = m\left( \sum_{k=i-1-n}^{k=i-1} \nabla \mathbf{d}_k \right) / n$$

The multiple coefficient m and the cutting point n may be suitably set according to the image being processed, with their numerical values being about 5 for m and 10 for n, for example.

In this embodiment, as discussed above, whether or not to perform cutting is determined by comparing the gradient $\nabla d$ and the threshold $T_i$ calculated on the basis of the multiple coefficient m and the gradient average for n number of cutting points in the immediate vicinity, and using the result as the proportional change.

How the proportional change is calculated is not limited to the above, and any calculation formula may be used so long as the gradient change state can be confirmed.

Also, it is preferable if the threshold T is suitably varied according to the characteristics of the organ serving as the cutting object. This allows accidental cutting to be avoided more accurately.

In the cutting processing discussed above, a point having a proportional change over a specific threshold is deemed an invalid cutting point, and the depth controller 17 issues an instruction to the voxel label setting section 18. Consequently, updating of the voxel labels is halted, and cutting is not carried out. Thus, accidental cutting can be avoided when the depth detector 15 has detected a cutting point whose depth position changes suddenly due to operational error by the physician or other user.

Here, the phrase "cutting is performed" means that the voxel label setting section 18 updates the voxel labels and stores them in the voxel label storage section 11. That is, when cutting is not performed, the voxel labels do not change.

Therefore, when the cursor is moved over the liver 22, the system avoids accidentally cutting the backbone or the like located further in. In this case, an image in which part of the liver 22 has been cut is displayed according to how many times the mouse 4 has been moved to the left and right or up and down.

A state in which the liver 22 has been cut can be recognized from a change in the color of the liver 22 when information from the window coordinate acquisition section 20 is sent through the color information setting section 21 to the color information storage section 12.

The "color information setting section 21" here means a converter that employs what is known as a look-up table. That is, with the personal computer 1 in this embodiment, as discussed above, there is information about the points constituted by I (x, y, z, α), and different color information and brightness information are set ahead of time by the color information setting section 21 for the surface and the interior of the liver 22. Consequently, if user operation indicates cutting the liver 22 from the surface, the color of the cut portion C will be displayed as being clearly different from the surrounding color according to the degree of this cutting.

Then, in S10 and S11, the color information labels and voxel information (voxel label values) of the portion of the liver 22 (the cutting object) corresponding to the cut portion C are updated.

FIG. 6 shows an updated state of the color information labels and the voxel labels when cutting processing is performed, with the majority of the voxel labels 14 at the very top being in a "1" state, that is, the surface state of the liver 22. Also, in FIG. 6, portions marked with "0" indicate cut voxels.

In this embodiment, as discussed above, voxel labels corresponding to the liver 22 and the blood vessels 23 are stored in the first voxel label storage section 11a and 11b of the voxel label storage section 11, and the color information labels are stored in the first color information storage section 12a and 12b of the color information storage section 12.

Thus, when an instruction for cutting a part of the liver 22 by the mouse 4, etc is inputted, since the liver 22 is the cutting object, in S10 the voxel label setting section 18 updates the voxel label values for the cut portion C of the liver 22, and at the same time, in S11, updates the color information label of the cut portion C of the liver 22. Consequently, as shown in FIG. 7a, the interior of the liver 22 from which the cut portion C of the liver 22 has been resected is displayed on the screen of the display 2 in a slightly different color from that of the surface of the liver 22.

In this embodiment, if the cut portion C of the liver 22 includes the blood vessels 23 designated as noncutting objects in S5 above, the voxel labels of the blood vessels 23 shown in FIG. 7b are interchanged at the cut portion C, and are displayed in a portion of the cut portion C without undergoing cutting processing, as shown in FIG. 7c.

More specifically, in S12, for the blood vessels 23 designated as non-cutting objects, after cutting processing, the voxel information and color information at the cut portion C and the voxel information and color information of the blood vessels 23 are interchanged.

Consequently, only the portion of the liver 22 included in the cut portion C for which a cutting instruction was inputted is displayed in a cut state, and the blood vessels 23 designated as non-cutting objects are displayed in an uncut state. As a result, in resecting the part of the liver 22 that is a cutting object (the cut portion C), the physician or other person performing the surgery simulation can simultaneously share with a plurality of people information about where in the cut portion C the blood vessels 23 are located.

Also, in this embodiment, since different color information is set for the liver 22 (cutting object) and the blood vessels 23 (non-cutting objects), the location and shape off the blood vessels 23 displayed in red can be more accurately recognized, as shown in FIG. 7c.

Furthermore, in this embodiment, since the start and end of cutting are switched by clicking the mouse button on and off, for example, the physician or other user drags the mouse 4 with the mouse button clicked on, while looking at the 3D display screen, which allows the cutting of the intended partial region of the liver 22 to be carried out easily and continuously.

Also, in this embodiment, the updating of the memory 9 can be performed when the power to the personal computer 1 is off. When the physician or other user starts dragging the mouse 4 while holding down the mouse button, just the information in the volume rendering calculator 13 is updated in the memory 9, which provides the user with a visually interactive cutting function.

Here, volume labels that are being used are temporarily stored without updating the memory 9. When the user then releases the mouse button, the memory content that had been temporarily stored is reflected in the memory 9. Consequently, a display can be obtained in which the liver 22 (the cutting object) has been cut down to a certain depth from its surface in a single drag operation by the user, and display of an excessively cut state can be prevented.

Also, in this embodiment, the voxel labels are the same size as the initial voxel information, but to express more precise cutting, voxel labels may be produced in a smaller size. With this method, the voxel information is not directly edited, and time information is assigned to the voxel labels, which makes possible operations such as undo and redo.

Also, in this embodiment, surgical simulation can be performed merely by moving the mouse 4 in a planar fashion. Thus, the proper surgical simulation can be performed from this standpoint as well.

Other Embodiments

An embodiment of the present invention was described above, but the present invention is not limited to or by the above embodiment, and various modifications are possible without departing from the gist of the invention.

(A)

In the above embodiment, an example was described in which the present invention was realized as a cutting simulation device, but the present invention is not limited to this.

For example, the present invention may be realized as a cutting simulation program that causes a computer to execute the control method shown in FIGS. 5a and 5b.

Further, the present invention may be realized as a recording medium that stores this cutting simulation program.

(B)

In the above embodiment, an example was given in which the voxel information and color information of the cut portion C and the voxel information and color information of the blood vessels 23 were interchanged after cutting processing of the blood vessels 23 designated as the non-cutting objects, but the present invention is not limited to this.

For example, just the voxel information of the cut portion may be interchanged with the voxel information of the non-cutting objects.

However, as shown in FIG. 7c, in terms of making it easier to see the blood vessels that are the non-cutting objects, it is preferable also to interchange the color information of the non-cutting objects for which different color information is set from that of the cutting object.

(C)

In the above embodiment, an example was given in which an X-ray CT image was used as the tomographic image information for forming a 3D image, but the present invention is not limited to this.

For example, a 3D image may be formed using tomographic image information acquired by magnetic resonance imaging (MRI) that does not make use of radiation.

(D)

In the above embodiment, an example of the cutting simulation pertaining to the present invention was given in which the liver 22 was designated as the cutting object and the blood vessels 23 as the non-cutting objects, but the present invention is not limited to this.

For example, the cutting object may be the stomach, a lung, a kidney, the pancreas, the large intestine, the small intestine, the duodenum, or another organ, or bone. Alternatively, when the simulation involves cutting out a tumor or the like present near nerves or bone, then nerves or bone may be set as the non-cutting objects.

Also, if even the type of blood vessel is designated as a non-cutting object, it will also be possible to display in different colors so that arteries and veins can be distinguished, by suitably designating CT values.

(E)

In the above embodiment, an example was given in which brightness information and color information about the display object were both varied in the voxel labels 14 designated for cutting by the mouse 4, but the present invention is not limited to this.

For example, just brightness information or color information about the display object may be varied, or both.

(F)

In the above embodiment, the cutting amount (volume) inputted with the mouse 4 may be displayed on the display 2 as the output of the resection voxel label calculation and display unit 19, which calculates the volume of the cut voxels.

Alternatively, the cutting depth inputted with the mouse 4 may be displayed on the display 2.

(G)

In the above embodiment, an example was given in which a cutting simulation was performed while the user looked at a 3D image displayed on the screen of the display 2, but the present invention is not limited to this.

For example, a 2D tomographic image may be projected onto a 3D image indicating the results of volume rendering, and the cutting operation performed on the 2D tomographic image.

Here again, the cutting simulation is performed so that the cutting operation is reflected by the 3D image.

(H)

In the present invention, the color information setting section 21 may be provided that converts the voxel information stored in the voxel information storage section 10 into a 2D or 3D image and displays it on the display 2, and that changes the color information of the portion of the cut portion C displayed on the display 2 that is designated with the mouse 4.

That is, a color may be intentionally added to a portion that bothers the physician, for example, in the cutting object displayed on the display 2, and a grouping of voxel labels 14 may be stored in this state in the voxel label storage section 11.

Consequently, information to which color information has been added is reflected in a multipronged display extracted from this information. Thus, this portion of interest in the cutting object can be viewed stereoscopically from all around, and this cutting simulation can also be carried out.

(I)

In the present invention, it is also possible to simulate endoscopic surgery. In this case, the convergence characteristics of a fisheye lens or the like provided to an endoscope may be used as a coordinate conversion table in the volume rendering calculator 13.

(J)

In the present invention, it is also possible to produce a stereoscopic image by having a plurality of viewpoints, storing in a plurality of memories the output image of the volume rendering calculator 13 produced for each viewpoint, and displaying this output successively from the memories.

In this case, a liquid crystal goggles or the like that are synchronized to the image outputs may be used.

INDUSTRIAL APPLICABILITY

The cutting simulation device of the present invention has the effect of allowing an actual surgical procedure to be expressed in detail by visualizing the blood vessels, nerves, and so forth that are present inside the cutting object, and is therefore expected to have broad applicability as an apparatus for cutting simulation in surgical operations.

REFERENCE SIGNS LIST

| 1 | personal computer (cutting simulation device) |
|---|---|
| 2 | display (display unit) |
| 3 | keyboard (input section) |
| 4 | mouse (input section) |
| 5 | tablet (input section) |
| 6 | tomographic image information acquisition section |
| 7 | voxel information extractor |
| 8 | tomographic image information section |
| 9 | memory |
| 10 | voxel information storage section |
| 11 | voxel label storage section |
| 11a to 11c | first to third voxel label storage sections |
| 12 | color information storage section |
| 13 | volume rendering calculator |
| 14 | voxel label |
| 15 | depth detector |
| 16 | bus |
| 17 | depth controller |
| 18 | voxel label setting section (display controller) |
| 19 | resection voxel label calculation and display section |
| 20 | window coordinate acquisition section |
| 21 | color information setting section |
| 21 a to 21c | first to third color information setting sections |
| 22 | liver |
| 23 | blood vessel |
| C | cut portion |

**Claims**

1. A cutting simulation device, comprising:

   a tomographic image information acquisition section configured to acquire tomographic image information;
   a memory connected to the tomographic image information acquisition section, configured to store voxel information about the tomographic image information;
   a volume rendering calculator connected to the memory, configured to sample voxel information in a direction perpendicular to the line of sight, on the basis of the voxel information;
   a display unit configured to display calculation results from the volume rendering calculator;
   an input section with which instructions for cutting a cutting object displayed on the display unit is inputted; and
   a display controller configured to display on the display unit the status of the cutting object after cutting on the basis of the cutting instructions inputted with the input section, and displaying non-cutting objects included inside the cutting object, on the display unit in a pre-cut state even after cutting, even when included in a cut portion according to the cutting instructions.

2. The cutting simulation device according to Claim 1, wherein, if the non-cutting objects are included in the cut portion, the display controller switches the voxel data for the cut portion with voxel data for the non-cutting objects, and displays the result on the display unit.

3. The cutting simulation device according to Claim 1 or 2, wherein color information is added to the voxel information corresponding to the cutting object and the non-cutting objects in display on the display unit, and the display controller displays the non-cutting objects on the display unit in a different color from the color of the cutting object.

4. The cutting simulation device according to any of Claims 1 to 3, wherein a range of CT values indicating the extent of spatial X-ray absorption during X-ray irradiation of a subject is set for the cutting object and the non-cutting objects, and
the display controller displays the desired cutting object and non-cutting objects on the display unit on the basis of the CT values inputted via the input section.

5. The cutting simulation device according to any of Claims 1 to 4,
wherein the cutting object includes an organ or bone.

6. The cutting simulation device according to any of Claims 1 to 5,
wherein the non-cutting object includes blood vessels or nerves.

7. A cutting simulation program for causing a computer to execute a cutting simulation method comprising:

an acquisition step of acquiring tomographic image information;
a volume rendering step of sampling voxel information in a direction perpendicular to the line of sight, on the basis of voxel information about the tomographic image information;
a first display step of displaying calculation results from the volume rendering; and
a second display step of displaying on the display unit the status of the cutting object after cutting on the basis of the cutting instructions inputted with respect to the displayed cutting object, and displaying non-cutting objects included inside the cutting object, on the display unit in a pre-cut state even after cutting, even when included in a cut portion according to the cutting instructions.

FIG. 1

FIG. 2

EP 2 737 854 A1

11

first voxel label
storage section

— 11a

second voxel label
storage section

— 11b

third voxel label
storage section

— 11c

FIG. 3

12
12a — first color information storage section
12b — second color information storage section
12c — third color information storage section

FIG. 4

FIG. 5

Start

S1 — Input tomographic image

S2 — Extract voxel information

S3 — Volume rendering ◄— B

S4 — Display rendering image

S5 — Designate non-cutting objects

S6 — Cutting instructions — N

Y

A

(a) drawing process

A

S7 — Detect object depth

S8 — Is change in depth within specific range? — N

Y

S9 — Execute cutting

S10 — Update label values for liver at cut portion

S11 — Update color information labels for liver at cut portion

S12 — Interchange cut portion label values and blood vessel label values

B

(b) depth detection and cutting process

EP 2 737 854 A1

15

cutting labels

cutting information
(0: coordinates designated for cutting)

14

14

| 1 | 1 | 1 | 1 | 1 | 1 |
| 1 | 1 | 1 | 1 | 1 | 1 |
| 1 | 1 | 1 | 1 | 1 | 1 |
| 1 | 1 | 1 | 1 | 1 | 1 |
| 1 | 1 | 1 | 1 | 1 | 1 |
| 1 | 1 | 1 | 1 | 1 | 1 |

| 1 | 1 | 1 | 1 | 1 | 1 |
| 1 | 1 | 1 | 1 | 1 | 1 |
| 1 | 1 | 1 | 1 | 1 | 1 |
| 1 | 0 | 0 | 1 | 1 | 1 |
| 1 | 0 | 0 | 1 | 1 | 1 |
| 1 | 1 | 1 | 1 | 1 | 1 |

color information labels

| 1 | 1 | 1 | 1 | 1 | 1 |
| 1 | 1 | 1 | 1 | 1 | 1 |
| 1 | 1 | 1 | 1 | 1 | 1 |
| 1 | 1 | 1 | 1 | 1 | 1 |
| 1 | 1 | 1 | 1 | 1 | 1 |
| 1 | 1 | 1 | 1 | 1 | 1 |

| 1 | 1 | 1 | 1 | 1 | 1 |
| 1 | 1 | 1 | 1 | 1 | 1 |
| 1 | 1 | 1 | 1 | 1 | 1 |
| 1 | n | n | 1 | 1 | 1 |
| 1 | n | n | 1 | 1 | 1 |
| 1 | 1 | 1 | 1 | 1 | 1 |

volume data: voxel grouping

0: default
1: nerve
2: blood vessel
3: muscle
:
n:

FIG. 6

(a) Typical display

(c) Present invention display

(b) Display of blood vessel portion

FIG. 7

<table>
<tr><td colspan="2" align="center">**INTERNATIONAL SEARCH REPORT**</td><td>International application No.<br>PCT/JP2012/004378</td></tr>
</table>

**A. CLASSIFICATION OF SUBJECT MATTER**
*A61B6/03*(2006.01)i, *G06T1/00*(2006.01)i

According to International Patent Classification (IPC) or to both national classification and IPC

**B. FIELDS SEARCHED**

Minimum documentation searched (classification system followed by classification symbols)
A61B6/03, G06T1/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2003-010172 A (Hitachi Medical Corp.),<br>14 January 2003 (14.01.2003),<br>entire text; all drawings<br>(Family: none) | 1-7 |
| A | JP 2003-339644 A (Hitachi Medical Corp.),<br>02 December 2003 (02.12.2003),<br>entire text; all drawings<br>(Family: none) | 1-7 |
| A | JP 9-073556 A (Toshiba Corp.),<br>18 March 1997 (18.03.1997),<br>entire text; all drawings<br>(Family: none) | 1-7 |

☒ Further documents are listed in the continuation of Box C.    ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>31 July, 2012 (31.07.12) | Date of mailing of the international search report<br>07 August, 2012 (07.08.12) |
|---|---|
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2012/004378

C (Continuation).   DOCUMENTS CONSIDERED TO BE RELEVANT

| Category* | Citation of document, with indication, where appropriate, of the relevant passages | Relevant to claim No. |
|---|---|---|
| A | JP 64-037678 A  (Toshiba Corp.),<br>08 February 1989 (08.02.1989),<br>entire text; all drawings<br>(Family: none) | 1-7 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H5123327 B

- JP 2003091735 A